# EUROPÄISCHE PATENTSCHRIFT

(11) **EP 0 714 447 B1**
(45) Veröffentlichungstag und Bekanntmachung des Hinweises auf die Patenterteilung: **20.05.1998**
(21) Anmeldenummer: 94925459.3
(22) Anmeldetag: 17.08.1994
(51) Int. Cl.: C12P 7/40

(54) **VERFAHREN ZUR HERSTELLUNG VON (L)-2-CHLORPROPIONSÄURE UND DEREN SALZEN**
PROCESS FOR PREPARING (L)-2-CHLORPROPIONIC ACID AND ITS SALTS
PROCEDE DE PREPARATION D'ACIDE CHLOROPROPIONIQUE-2-(L) ET DE SES SELS

(30) Priorität: 23.08.1993 DE 4328231
(43) Veröffentlichungstag der Anmeldung: 05.06.1996
(73) Patentinhaber: BASF AKTIENGESELLSCHAFT, 67056 Ludwigshafen (DE)
(72) Erfinder: LADNER, Wolfgang, D-67136 Fussgönheim (DE); RETTENMAIER, Hansjörg, D-67269 Grünstadt (DE); ZIPPERER, Bernhard, D-67246 Dirmstein (DE); HANSEN, Hanspeter, D-67061 Ludwigshafen (DE)
(86) Internationale Anmeldenummer: EP9402738
(87) Internationale Veröffentlichungsnummer: WO9506130

(56) Entgegenhaltungen:
- EP-A- 0 257 716
- EP-A- 0 511 526
- BIOTECHNOLOGY & BIOINGE- NEERING, Band 30, Nr. 5, ver!ffentlicht 1987, 05 Oktober S.K. DAHOD et al. "Carbon Tetrachloride-Promoted Stereo selective Hydrolysis o f Methyl-2-Chloroproprionate by Lipase", Seiten 995-999,
- PATENT ABSTRACTS OF JAPAN, unexamined applications, C Field, Band 10, Nr. 296, ver!ffentlicht 1986, 08 Oktober THE PATENT OFFICE JAPANESE GOVERNMENT Seite 52 C 377; & JP-A-61 111 699 (TORAY IND.)

## Beschreibung

Die vorliegende Erfindung betrifft ein neues Verfahren zur Herstellung von (L)-2-Chlorpropionsäure und deren Alkalimetall-, Erdalkalimetall- oder Ammoniumsalzen.

Aus JP 57 094 295, JP 62 205 797, EP-A 196 625, JP 61 111 699 und Appl. Biochem. Biotechnol. 9(3), (1984) 255 ist bekannt, racemische 2-Chlorpropionsaureester mittels Enzymen enantioselektiv zu spalten. Nachteilig hierbei ist allerdings neben der maximal möglichen Ausbeute von lediglich 50 % die ungenügende Enantiomerenreinheit der gebildeten 2-Chlorpropionate.

Ferner wird in der EP-A 257 716 ein kontinuierliches Verfahren gelehrt, bei dem D,L-2-Brompropionsäuremethylester als Racemat eingesetzt und in einem 2-Phasensystem mittels Candida cylindracea Lipase in L-(-)-2-Brompropionsäuremethylester umgewandelt wird. Bei diesem Verfahren werden jedoch keine Hydrolyseprodukte isoliert.

Des weiteren beschreiben S.K. Dahod und P. Sinta-Mangano in Biotechnol. Bioeng. 30(8), 995 (1987) die Lipase-katalysierte Hydrolyse von L-2-Chlorpropionsäuremethylester in Gegenwart von Kohlenstofftetrachlorid. Nachteilig hierbei ist neben der Verwendung eines chlorierten Lösungsmittels die geringe Ausbeute von ca. 30 % bei nur 95 % Enantiomerenreinheit.

Außerdem ist der EP-A 511 526 eine Verfahren zur enzymatischen Hydrolyse von racemischen 2-Chlorpropionsaureestern in einem 2-Phasensystem, das aus Wasser und einem mit Wasser weitgehend unmischbaren organischen Lösungsmittel für den Ester besteht, zu entnehmen. Dieses Verfahren ist sehr aufwendig, denn die organische Phase muß mehrmals abgetrennt, mit der Hydrolase in Kontakt gebracht, und wieder rückgeführt werden. Da trotz relativ langer Reaktionszeiten nur ein unvollständiger Umsatz stattfindet und keine enantiomerenreinen Produkte erhalten werden, ist diese Methode für die technische Herstellung von (L)-2-Chlorpropionsäure und deren Natriumsalz ungeeignet.

(L)-Natrium-2-chlorpropionat und (L)-2-Chlorpropionsäure wurden bisher durch alkalische Hydrolyse von (L)-2-Chlorpropionsäureisobutylester hergestellt. Hierbei fällt jedoch oft ein qualitativ minderwertiges Produkt an (ca. 90-95 % Enantiomerenüberschuß), das zudem noch 5-10 % Milchsäure als Nebenprodukt enthält.

Aufgabe der vorliegenden Erfindung war daher ein einfaches Verfahren zur Herstellung von (L)-2-Chlorpropionsäure und (L)-Natrium-2-chlorpropionat mit möglichst hoher chemischer und optischer Reinheit (mindestens ca. 98 % Enantiomerenüberschuß und weniger als 1% Milchsäure).

Demgemäß wurde ein Verfahren zur Herstellung von (L)-2-Chlorpropionsäure und deren Alkalimetall-, Erdalkalimetall- oder Ammoniumsalzen gefunden, welches dadurch gekennzeichnet ist, daß man L-Chlorpropionsäureisobutylester bei einem pH-Wert von 4 bis 8 in Gegenwart einer Lipase aus Pseudomonas spec. DSM 8246*) hydrolysiert und das optisch aktive Reaktionsprodukt entweder direkt oder nach Überführung des Salzes in die Säure in an sich üblicher Weise aus dem Reaktionsgemisch isoliert oder in situ weiter umsetzt.
*) DSM = Deutsche sammlung von Mikroorganismen und Zellkulturen GmbH, Mascheroder Weg 1B, 38124 Braunschweig

Die Herstellung von L-Chlorpropionsäureisobutylester erfolgt vorteilhaft ausgehend von D-Milchsäure, die beispielsweise biotechnologisch nach einem aus der EP-A 069 291 bekannten Verfahren hergestellt werden kann. Dabei wird eine Mischung aus wässriger Glucoselösung, autolysierter Bierhefe, Vitaminen, katalytischen Mengen an Phosphorsäure und einem Puffer für die entstehende Milchsäure, z.B. Calciumcarbonat, unter Zusatz von Milchsäurebakterien bei etwa 45 °C fermentiert. Der pH-Wert der Fermentationsbrühe liegt vorzuzgsweise bei 4 bis 6. Unter Ausgasen von Kohlendioxid entsteht ein Lactat, das durch Zugabe einer Säure, bevorzugt konzentrierter wässriger Schwefelsäure, in D-Milchsäure übergeführt wird. Die D-Milchsäure wird anschließend mit Isobutanol extrahiert. Danach wird die erhaltene D-Milchsäure-Lösung aufkonzentriert. Ein Teil der Milchsäure wird dabei bereits verestert. Die restliche Menge der Milchsäure wird unter saurer Katalyse verestert, wobei als Säure z.B. Schwefelsäure geeignet ist (vgl. z.B. EP-A 287 426, DE-A 32 14 697 und DE-A 34 33 4001. Anschließend wird das gebildete Reaktionswasser zusammen mit nicht umgesetztem Isobutanol abdestilliert.

Der erhaltene D-Milchsäureisobutylester kann ohne weitere Reinigung auf bekannte Weise chloriert werden (vgl. z.B. EP-A 401 104, JP 61 057 534 (1986), JP 02 104 560 (1990), JP 61 068 445 (1986) und FR-A 24 59 221. Die Chlorierung erfolgt vorzugsweise mit Thionylchlorid in Gegenwart eines Katalysators, z.B. N,N-Dimethylformamid, wobei Inversion am asymmetrischen C-Atom erfolgt.

Das Rohprodukt wird von den Schwersiedern abgetrennt und anschließend destillativ gereinigt.

Nach der vorstehend beschriebenen Weise kann der L-Chlorpropionsäureisobutylester mit einer chemischen Reinheit von ca. 98 - 99 % erhalten werden. Die optische Reinheit (L:D) beträgt etwa 98 - 100 %.

Die Lipase läßt sich aus Pseudomonas spec. DSM 8246 gewinnen, indem man das Bakterium in einem Nährmedium züchtet und das Enzym aus der Kulturbrühe isoliert. Geeignet sind Nährmedien, die Kohlenstoffquellen, Stickstoffquellen, anorganische Salze und gegebenenfalls geringe Mengen an Spurenelementen und Vitaminen enthalten. Als Stickstoffquellen können anorganische oder organische Stickstoffverbindungen oder Materialien, die diese Verbindungen enthalten, verwendet werden. Beispiele sind: Ammoniumsalze, Nitrate, Maisquellwasser, Hefeautolysat, Hefeextrakt und hydrolisiertes Casein. Als Kohlenstoffquellen können Zucker wie Glucose, Polyole wie Glycerin oder auch organische Säuren wie Citronensäure oder Fettsäuren verwendet werden. Besonders geeignet als Kohlenstoffquelle sind pflanzliche Öle wie Sojaöl, Leinöl oder Olivenöl. Beispiele für die anorganischen Salze sind die Salze von Calcium, Magnesium, Mangan, Kalium, Zink, Kupfer, Eisen und anderen Metallen. Als Anionen der Salze sind besonders Phosphat und Nitrationen zu nennen.

Bevorzugte Züchtungstemperaturen liegen bei 25 bis 33°C. Der pH-Wert des Mediums wird während der Fermentation mittels Mineralsäuren wie 2n-Schwefelsäure oder Basen wie Ammoniak bei 6 bis 7,5, vorzugsweise bei 6,5 bis 7, gehalten. Die Kultur wird als Submerskultur unter intensiver Belüftung und Rühren ausgeführt. Es wird bis zur Konstanz der Aktivität bei zwei aufeinanderfolgenden Enzymaktivitätsmessungen im Abstand von drei Stunden fermentiert. Im allgemeinen ist eine Inkubationsdauer von 40 bis 60 Stunden ausreichend. Auf diese Weise lassen sich Enzymausbeuten von 50 bis 500 mg pro 1 Kulturbrühe erzielen.

Das Enzym wird aus der Kulturbrühe in üblicher Weise isoliert. Um die Mikroorganismen und unlösliches Material abzutrennen wird die Brühe zentrifugiert oder filtriert. Aus der gesammelten flüssigen Phase erhält man dann die Lipase entweder durch Ausfällen mit einem mit Wasser mischbaren organischen Lösungsmittel, z.B. Aceton oder einem niedrigen Alkohol, oder durch Zusatz eines Salzes, insbesondere Ammoniumsulfat.

Zur Erhöhung der spezifischen Aktivität und zur weiteren Abreicherung von Verunreinigungen der erhaltenen Roh-Lipase ist es möglich, diese wieder aufzulösen und erneut auszufallen, beispielsweise durch Zusatz von Lösungsmitteln oder von Salz (fraktionierte Ausfällung). Die Roh-Lipase kann aber auch mittels Querstromfiltrationen der das Enzym enthaltenden Lösung über geeignete Ultrafiltrationsmembranen gereinigt werden. Bei dieser Methode passieren niedermolekulare Verunreinigungen die Membran während das Enzym zurückgehalten wird.

Besonders vorteilhaft an dem vorstehenden Verfahren ist, daß die Lipase-Konzentration der erhaltenen wässrigen Lösung sehr hoch ist. Sie liegt normalerweise zwischen etwa 5 und 30 g/l.

Die Lipase-Lösung kann direkt für das vorliegende Verfahren verwendet werden. Es besteht jedoch auch die Möglichkeit, die Lipase mittels festem Träger immobilisert einzusetzen. Als feste Träger eignen sich die hierfür üblichen inerten Trägermaterialien (vgl. z.B. Enzyme and Microbial. Technology 14, 426 (1992)}.

Das vorliegende Verfahren wird vorteilhaft in einem 2-Phasensystem aus wässriger Enzymlösung und L-Chlorpropionsäureisobutylester, der zusammen mit dem während der Reaktion entstehenden Isobutanol die zweite Phase bildet, durchgeführt.

Der organischen Phase kann zusätzlich ein mit Wasser weitgehend unmischbares Lösungsmittel, z.B. ein Kohlenwasserstoff wie n-Hexan, ein Chlorkohlenwasserstoff oder ein Ether zugesetzt werden.

Die Hydrolyse von L-Chlorpropionsäureisobutylester erfolgt in einem pH-Bereich von etwa 4 bis 8, vorzugsweise 5 bis 7. Der pH-Wert wird üblicherweise vor Zugabe der Lipase eingestellt und während der Reaktion etwa konstant gehalten, indem man normalerweise eine Base kontinuierlich oder portionsweise hinzufügt. Es ist jedoch auch möglich, in einem geeigneten gepufferten System zu arbeiten und die gesamte Menge an Base (z.B. Natriumhydrogencarbonat) vorzulegen.

Als Basen eignen sich hierfür beispielsweise Alkalimetallhydroxide wie Natrium- und Kaliumhydroxid, Alkali- und Erdalkalimetallhydrogencarbonate wie Natriumhydrogencarbonat, Kaliumhydrogencarbonat und Calciumhydrogencarbonat, Alkali- und Erdalkalimetallcarbonate wie Natriumcarbonat, Kaliumcarbonat und Calciumcarbonat, oder tertiäre Amine wie Triethylamin.

Da aus Kostengründen die Herstellung des Natriumsalzes der (L)-2-Chlorpropionsäure bevorzugt ist, verwendet man eine entsprechende Natriumverbindung wie Natriumhydroxid, Natriumhydrogencarbonat und Natriumcarbonat als Base.

Im allgemeinen liegt die Reaktionstemperatur zwischen 5 und 60 °C, bevorzugt zwischen 20 und 40 °C.

Die Reaktion kann bei Normaldruck, unter dem Eigendruck der Reaktionsmischung oder unter vermindertem Druck ausgeführt werden.

Eine in Bezug auf den Reaktionsverlauf besonders vorteilhafte Ausführungsform besteht darin, das während der Reaktion gebildete Isobutanol laufend zu entfernen, vorzugsweise destillativ unter reduziertem Druck.

Das optisch aktive Reaktionsprodukt kann nun in an sich üblicher Weise aus dem Reaktionsgemisch isoliert oder in situ weiter umgesetzt werden. Bezüglich der weiteren Umsetzung sei beispielhaft auf die Ausführungen in den Druckschriften GB-A 20 54 570, DE-A 30 24 265 und EP-A 009 285 verwiesen.

Zwecks Isolierung des optisch aktiven Reaktionsproduktes wird die organische Phase zusammen mit der Lipase abgetrennt. Danach entfernt man den in der wässrigen Phase gelösten Anteil an Isobutanol, zweckmäßigerweise destillativ (z.B. bei 50-60 °C und 20-50 mbar Druck). Die wässrige (L)-Natrium-2-chlorpropionat-Lösung hat danach erfahrungsgemäß einen Restgehalt an Isobutanol von weniger als 0,3 gew.-%. Da sie bei 20-25 °C jedoch nicht über längere Zeit gelagert werden kann empfiehlt es sich, bis zur Weiterverarbeitung zu kühlen oder die Lösung anzusauern und gewünschtenfalls auf (L)-2-Chlorpropionsäure hin aufzuarbeiten. Durch Aufkonzentration der etwa 30 gew.-%igen wässrigen (L)-Natrium-2-chlorpropionat-Lösung auf 50-70 gew.-% und anschließender Kühlung auf (-20) bis (-50) °C erhält man beispielsweise eine Kristallsuspension, die mehrere Wochen lang stabil ist.

Das (L)-Natrium-2-chlorpropionat läßt sich auf an sich bekannte Weise isolieren, z.B. durch die vorstehend erwähnte Tieftemperaturkristallisation, aber auch durch Sprüh- oder Gefriertrocknung. Im Falle der Sprühtrocknung erhält man beispielsweise mit einer Eintrittstemperatur von 200 °C und einer Ausgangstemperatur des Feststoffes von 80-90 °C das (L)-Natrium-2-chlorpropionat als trockenes, rieselfähiges Pulver mit einem Restwassergehalt von 0,2 bis 0,7 %. Die optische Aktivität bleibt in jedem Fall vollständig erhalten.

Durch Ansäuern der wässrigen (L)-Natrium-2-chlorpropionat-Lösung, z.B. mit Schwefelsäure, auf einen pH-Wert unter 2 erhält man die freie (L)-2-Chlorpropionsäure. Letztere kann aus der wässrigen Phase mittels geeigneter organischer Lösungsmittel, die eine gute Löslichkeit für 2-Chlorpropionsäure aufweisen und mit Wasser nur wenig mischbar sind, extrahiert werden. Hierfür kommen beispielsweise Ether wie Methyl-tert.-butylether oder chlorierte Kohlenwasserstoffe wie Dichlormethan und 1,2-Dichlorethan in Betracht.

Nach Entfernen des organischen Lösungsmittel kann die (L)-2-Chlorpropionsaure dann auf übliche Weise gereinigt werden, zweckmäßigerweise destillativ bei reduziertem Druck (bei 12 mbar Druck liegt die Kopftemperatur beispielsweise bei ca. 80 °C).

Die (L)-2-Chlorpropionate und die (L)-2-Chlorpropionsäure sind wichtige Zwischenprodukte für Pflanzenschutzmittel und Pharmaka. Insbesondere eignen sie sich zur Herstellung von D-2-Phenoxypropionsäure (vgl. z.B. DE-A 15 43 841), die biotechnologisch in R-2-(4-Hydroxyphenoxy)-propionsaure übergeführt werden kann (vgl. hierzu z.B. WO 90/11362 und EP-A 465 494). R-2-(4-Hydroxyphenoxy)-propionsäure dient letztlich als Ausgangsprodukt bei der Herstellung herbizid wirksamer Aryloxyphenoxypropionsäure-Derivate.

### Herstellungsbeispiele

### Beispiel 1

Herstellung und Reinigung der Lipase aus Pseudomonas spec. DSM 8246:

Zur Züchtung des Mikroorganismus Pseudomonas spec. DSM 8246 wurde folgendes Medium verwendet:

| | | |
|---|---|---|
| KH₂PO₄ | 20 | g/l |
| Na₂HPO₄ | 10 | g/l |
| MgSO₄ | 5 | g/l |
| CaCl₂ x 2H₂O | 3 | g/l |
| FeSO₄ x 7H₂O | 0,5 | g/l |
| MnSO₄ x 4H₂O | 0,005 | g/l |
| CoCl₂ x 6H₂O | 0,005 | g/l |
| CuSO₄ x 5H₂O | 0,005 | g/l |
| ZnSO₄ x 7H₂O | 0,005 | g/l |
| Hefeextrakt | 5 | g/l |

Als Kohlenstoffquelle diente raffiniertes Sojaöl, welches mit einer konstanten Zudosierrate von 1 g/l x h zugepumpt wurde. Der pH-Wert wurde über die gesamte Fermentationszeit mit Hilfe von 2 n H₂SO₄ und 25 %igem NH₄OH konstant bei pH 6,5 gehalten.

Die Impfkultur wurde durch Impfen von 400 ml Nutrient-broth-Medium pH 6,5 mit dem Mikroorganismus Pseudomonos spec. DSM 8246 erhalten.

Die Impfkultur wurde 10 Std. bei 30°C auf einer Schüttelvorrichtung inkubiert.

Das Medium wurde bei 30°C und einem pH-Wert von 6,5 mit 5 Volumenteilen der Impfkultur pro 100 Volumenteile Medium beimpft. Die Hauptzüchtung wurde bei 30°C in 10 l-Rührfermentern mit 8 l Inhalt durchgeführt. Die Rührgeschwindigkeit der eingebauten Blattrührer betrug 1000 Umdrehungen pro Minute, die Belüftungsrate lag bei einem Volumen Luft pro Minute und Volumen Fermentationsbrühe. Nach 60 Std. zeigte die Fermentationsbrühe bei zwei aufeinanderfolgenden Aktivitätsmessungen eine konstante Aktivität von 300 Enzymeinheiten pro ml nach F.I.P. (= Federation International Pharmaceutique; zur Methode siehe z.B. R. Ruyssen u. A. Lauwers, Pharmaceutical Enzymes, E. Story-Scientia P.V.B.A., Scientific Publishing Company, Gent/Belgien, 1978, S. 78-82).

Daraufhin wurde die Fermentation abgebrochen und die gebildete Lipase folgendermaßen aus der Fermentationsbrühe isoliert:

Der Fermenteraustrag wurde mit n-Propanol auf einen Volumenanteil von 65 % Alkohol verdünnt. Biomasse und ausgefüllte Nebenprodukte wurden durch Zentrifugation abgetrennt. Die klare, alkoholische Enzymlösung wurde unter Vakuum auf ein Drittel des Ausgangsvolumens eingeengt. Obwohl bereits zur Hydrolyse von L-Chlorpropionsäureisobutylester geeignet, wurde diese Enzymlösung zur weiteren Aktivitatssteigerung im Diafiltrationsmodus (Cellulosetriacetat-Querstrom-Filtrationsmodule, Trenngrenze 20.000 nominelles Mol.-Gewicht, Fa. Sartorius, Göttingen) mit drei Volumina Wasser gewaschen und danach per Filtration auf ein Viertel des Ausgangsvolumens eingeengt. Aus diesem Enzymkonzentrat wurde die Lipase ausgefällt, indem n-Propanol bis zu einem Volumenanteil von 85 % zugegeben wurde. Der die Lipaseaktivität enthaltende Niederschlag wurde durch Zentrifugation geerntet und in einer 65 Volumenteile n-Propanol enthaltendem, wäßrigen Lösung aufgenommen. Das Gewichtsverhältnis Niederschlag zu n-Propanol/Wasser-Gemisch lag bei 1 zu 10.

Durch Zentrifugation wurde ungelöster Niederschlag abgetrennt. Aus dem klaren Überstand der Zentrifugation wurde die Lipase ausgefällt, indem der n-Propanolanteil auf 80 Volumenanteile erhöht wurde. Der Niederschlag wurde durch Zentrifugation geerntet und gefriergetrocknet. Das so erhaltene Enzympulver zeigte eine spezifische Aktivität von 7100 Enzymeinheiten nach F.I.P. pro Milligramm Protein.

### Beispiel 2

Verseifung von L-Chlorpropionsäureisobutylester mittels Lipase (erfindungsgemäß):

Eine Suspension aus 200 g L-Chlorpropionsäureisobutylester in 400 g Wasser wurde bei 20-25 °C intensiv gerührt und mit 25 gew.-%iger wässriger Natronlauge neutralisiert (pH-Wert = 7,5). Danach gab man 500 mg Lipase (aus Pseudomonas spec. DSM 8246; Aktivität ca. 400 U/mg) zu und hielt den pH-Wert der Reaktionsmischung durch kontinuierliche Zugabe von 10 normaler wässriger Natronlauge konstant bei 7,5. Nach 6¼ Std. waren 97,3 % der theoretisch benötigten Menge an Natronlauge verbraucht. Um die Reaktion abzubrechen wurde die Isobutanol-Phase zusammen mit der Lipase abgetrennt. Aus der wässrigen Phase erhielt man durch Gefriertrocknung (L)-Natrium-2-chlorpropionat mit einer Enantiomerenreinheit von 99,2 %. Ausbeute: 95,8 %. Die Verunreinigung durch Milchsäure lag unter 0,05 %.

### Beispiel 3

Verseifung von L-Chlorpropionsäureisobutylester mittels immobilisierter Lipase (erfindungsgemäß):

Eine Suspension aus 1000 g (6,08 mol) L-Chlorpropionsäureisobutylester in 2000 g Wasser wurde bei 20-25 °C intensiv gerührt und mit 25 gew.-%iger wässriger Natronlauge versetzt, bis der pH-Wert bei 5-6 lag. Danach wurden 2,5 g Lipase (Aktivität ca. 400 U/mg; aus Pseudomonas spec. DSM 8246; Lipase immobilisiert auf Polypropylen-Pulver "Accurel® EP100" von Akzo, Körnung 200-1000 µ) zugegeben, wobei man den pH-Wert der Reaktionsmischung durch kontinuierliche Zugabe von 25 gew.-%iger Natronlauge konstant (zwischen 5 und 6) hielt. Nachdem 98 % der theoretisch benötigten Menge an Natronlauge verbraucht waren (nach ca. 18 Std.) filtrierte man die Lipase ab. Die organische Phase des Filtrats wurde abgetrennt. Aus der wässrigen Phase wurde bei 60°C und 50 mbar mittels Dünnschichtverdampfer gelöstes Isobutanol als Azeotrop mit Wasser abdestilliert. Man erhielt 2340 g einer ca. 30 gew.-%igen Lösung von L-Natriumchlorpropionat in Wasser (Ausbeute: ca. 88 %).

450 g dieser Lösung wurden mit ca. 60 g konz. Schwefelsäure auf einen pH-Wert von etwa 1 angesäuert. Nach Zugabe von ca. 200 ml Methyl-tert.-butylether wurde die organische Phase abgetrennt. Die wässriger Phase wurde noch dreimal mit je 200 ml Methyl-tert.-butylether extrahiert. Die vereinigten organischen Phasen wurden über Natriumsulfat getrocknet und bei 300 mbar/50°C eingeengt, wonach man den Rückstand über eine 30 cm Vigreux-Kolonne destillierte. Man erhielt 84 g L-Chlorpropionsäure als farblose Flüssigkeit vom Sdp. 82°C/13 mbar, die eine chemische Reinheit von mehr als 99% und eine optische Reinheit von L:D = 99:1 hatte.

1500 g der obigen Lösung von L-Natriumchlorpropionat in Wasser wurden auf einem Sprühturm bei 200°C Eingangstemperatur sprühgetrocknet. Bei einer Austrittstemperatur von 83°C erhielt man 480 g festes L-Natriumchlorpropionat als weißes Pulver mit einem Rest-Wassergehalt von 0,4 % und einer optischen Reinheit von 99:1 (L:D).

### Beispiel 4

Verseifung von L-Chlorpropionsäureisobutylester mittels Lipase unter laufender Entfernung von Isobutanol (erfindungsgemäß)

Eine Mischung aus 164,5 g (1,0 mol) L-Chlorpropionsäureisobutylester und 330 g Wasser wurde bei 35-40 °C unter Rühren mit 25 gew.-%iger wässriger Natronlauge versetzt, bis der pH-Wert bei 5-6 lag. Anschließend versetzte man das Gemisch mit 5,0 ml einer etwa 5 gew.-%igen Lipase-Lösung (Aktivität ca. 100000 U/ml). Die Umsetzung erfolgte bei 35-40 °C, wobei der pH-Wert der Reaktionsmischung.durch Zudosieren von 25 gew-%iger Natronlauge bei 5-6 gehalten wurde. Nachdem ca. 20 % der berechneten Menge an Natronlauge verbraucht waren, reduzierte man den Druck langsam auf 80 mbar, bis die Reaktionsmischung siedete. Das farblose Destillat mit einem Siedpunkt von 37 °C bei 80 mbar bestand aus Wasser, Isobutanol und geringen Mengen an L-Chlorpropionsäureisobutylester *). Der vollständige Umsatz an Ausgangsester (nach etwa 2 Stunden) war an einem starken Anstieg des pH-Wertes der Reaktionsmischung nach jedem weiteren Tropfen Natronlauge zu erkennen. Die Zugabe der Natronlauge wurde daraufhin sofort beendet. Anschließend rührte man einige Minuten, bis der pH-Wert wieder bei etwa 6 lag. Man erhielt 507 g einer 23 %igen L-Natrium-2-chlorpropionat-Lösung, die weniger als 0,5 % Isobutanol und maximal 0,05 % L-Chlorpropionsäureisobutylester enthielt. Ausbeute: 89 % (bezogen auf eingesetzten L-Chlorpropionsäureisobutylester).
*) 2-Phasen: 39 g einer wässrigen Phase aus 88 % Wasser und 12 % Isobutanol; 73 g einer organischen Phase aus 76 % Isobutanol, 12 % L-Chlorpropionsäureisobutylester und 12 % Wasser

### Beispiel 5 (= Vergleichsbeispiel)

Verseifung von (L)-2-Chlorpropionsäureisobutylester mit Natronlauge (ohne Lipase)

In ein 6 l Doppelmantel-Reaktionsgefäß wurden unter Rühren 2467 g (15 mol) L-CIB (Enantiomerenreinheit mindestens 99 %) und 1,6 1 Wasser gegeben. Zu der erhaltenen Suspension, die einen pH-Wert von etwa 2 hatte, gab man bei 40-45 °C insgesamt 1200 g (15 mol) 50 gew.-%ige wässrige Natronlauge. Die Zugabe erfolgte dabei nach Maßgabe des Verbrauchs an Natronlauge so, daß der pH-Wert konstant bei 12,3 lag. Nach beendeter Zugabe (ca. 2 Stunden) wurde die Reaktionsmischung mit 20 gew.-%iger Salzsäure neutralisiert (pH = 7-8). Anschließend destillierte man bei 60 mbar Druck und ca. 40 °C ein Azeotrop aus Isobutanol und Wasser ab, wobei während der Destillation noch etwa 500 ml Wasser zusätzlich vorgelegt wurden. Nach etwa 100 min waren ca. 2000 g Azeotrop abdestilliert. Der Rückstand wurde wie üblich auf das Produkt hin aufgearbeitet. Ausbeute: etwa 96 %; optischen Reinheit: 95,5:4,5 (L:D).

## Patentansprüche

1. Verfahren zur Herstellung von (L)-2-Chlorpropionsäure und deren Alkalimetall-, Erdalkalimetall- oder Ammoniumsalzen, dadurch gekennzeichnet, daß man L-Chlorpropionsäureisobutylester bei einem pH-Wert von 4 bis 8 in Gegenwart einer Lipase aus Pseudomonas spec. DSM 8246 hydrolysiert und das optisch aktive Reaktionsprodukt entweder direkt oder nach Überführung des Salzes in die Säure in an sich üblicher Weise aus dem Reaktionsgemisch isoliert oder in situ weiter umsetzt.

2. Verfahren nach Anspruch 1, dadurch gekennzeichnet, daß man die Reaktion bei einer Temperatur von 5 bis 60°C vornimmt.

3. Verfahren nach Anspruch 1, dadurch gekennzeichnet, daß man den pH-Bereich während der Reaktion durch Hinzufügen einer Base konstant hält.

4. Verfahren nach Anspruch 1, dadurch gekennzeichnet, daß man die Reaktion unter laufender Entfernung des hierbei entstehenden Isobutanols vornimmt.

5. Verfahren nach Anspruch 1, dadurch gekennzeichnet, daß die Lipase an einem festen Träger immobilisiert vorliegt.

6. Verfahren nach Anspruch 1, dadurch gekennzeichnet, daß man das optisch aktive Reaktionsprodukt aus dem nach der Hydrolyse anfallenden Reaktionsgemisch isoliert, indem man
a) die Lipase und die organische Phase abtrennt,
b) die wässrige Phase weitgehend von gelöstem Isobutanol befreit und
c) das L-2-Chlorpropionat isoliert oder mittels Säure zu L-Chlorpropion-säure protoniert und diese in ein inertes organisches Lösungsmittel überführt und aus diesem isoliert.

## Claims

1. A process for the preparation of (L)-2-chloropropionic acid and its alkali metal, alkaline earth metal or ammonium salts, which comprises hydrolyzing isobutyl L-chloropropionate at a pH of from 4 to 8 in the presence of a lipase from Pseudomonas spec. DSM 8246 and isolating the optically active reaction product from the reaction mixture either directly or after conversion of the salt into the acid in a conventional way, or further reacting it in situ.

2. A process as claimed in claim 1, wherein the reaction is carried out at from 5 to 60°C.

3. A process as claimed in claim 1, wherein the pH range is kept constant during the reaction by adding a base.

4. A process as claimed in claim 1, wherein the reaction is carried out with continuous removal of the isobutanol produced thereby.

5. A process as claimed in claim 1, wherein the lipase is immobilized on a solid carrier.

6. A process as claimed in claim 1, wherein the optically active reaction product is isolated from the reaction mixture resulting after the hydrolysis by
a) removing the lipase and the organic phase,
b) substantially removing dissolved isobutanol from the aqueous phase and
c) isolating the L-2-chloropropionate or protonating it with acid to give L-chloropropionic acid and transferring the latter into an inert organic solvent and isolating it therefrom.

## Revendications

1. Procédé de préparation de l'acide (L)-2-chloropropionique et de ses sels de métaux alcalins, de métaux alcalino-terreux ou d'ammonium, caractérisé par le fait que l'on hydrolyse le L-chloropropionate d'isobutyle à un pH de 4 à 8 en présence d'une lipase de Pseudomonas spec. DSM 8246, et on isole, de la manière habituelle, le produit de réaction possédant l'activité optique du mélange de réaction ou bien on le soumet à la concentration consécutive in situ soit directement, soit après conversion du sel en l'acide.

2. Procédé selon la revendication 1, caractérisé par le fait que l'on procède à la réaction à une température de 5 à 60°C.

3. Procédé selon la revendication 1, caractérisé par le fait que l'on maintient le pH constant pendant la réaction par addition d'une base.

4. Procédé selon la revendication 1, caractérisé par le fait que, dans le cours de la réaction, on élimine en permanence l'isobutanol formé.

5. Procédé selon la revendication 1, caractérisé par le fait que la lipase est immobilisée sur un support solide.

6. Procédé selon la revendication 1, caractérisé par le fait que l'on isole le produit de réaction possédant l'activité optique du mélange de réaction formé par hydrolyse
a) en séparant la lipase et la phase organique,
b) en débarrassant pratiquement totalement la phase aqueuse de l'isobutanol dissous et
c) en isolant le L-2-chloropropionate ou en le protonisant à l'aide d'un acide en l'acide L-chloropropionique, qui est transféré dans un solvant organique inerte et isolé de ce dernier.
